# EUROPÄISCHE PATENTSCHRIFT

(11) **EP 1 161 683 B1**
(45) Veröffentlichungstag und Bekanntmachung des Hinweises auf die Patenterteilung: **04.06.2003**
(21) Anmeldenummer: 00925109.1
(22) Anmeldetag: 11.03.2000
(51) Int. Cl.: G01N 33/50

(54) **CHEMOSENSITIVITÄTSBESTIMMUNG ÜBER PHOSPHATIDYLSERIN**
DETERMINATION OF THE CHEMOSENSITIVITY VIA PHOSPHATIDYL SERINE
DETERMINATION DE LA CHIMIOSENSIBILITE PAR L'INTERMEDIAIRE DE LA PHOSPHATIDYL-SERINE

(30) Priorität: 12.03.1999 DE 19910955; 30.04.1999 EP 99108496
(43) Veröffentlichungstag der Anmeldung: 12.12.2001
(73) Patentinhaber: Evotec Analytical Systems GmbH, 40699 Erkrath (DE)
(72) Erfinder: MEYER-ALMES, Franz, Josef, D-58636 Iserlohn (DE)
(74) Vertreter: Meyers, Hans-Wilhelm, Dr.Dipl.-Chem.
(86) Internationale Anmeldenummer: EP0002161
(87) Internationale Veröffentlichungsnummer: WO00054048

(56) Entgegenhaltungen:
- TOH H C ET AL: "Vinorelbine induces apoptosis and caspase-3 (CPP32) expression in leukemi and lymphoma cells: a comparison with vincristine." LEUKEMIA AND LYMPHOMA, (1998 SEP) 31 (1-2) 195-208., XP002118801 in der Anmeldung erwähnt
- KRAVTSOV, VLADIMIR D. ET AL: "Use of the microculture kinetic assay of apoptosis to determine chemosensitivities of leukemias" BLOOD (1998), 92(3), 968-980, XP002118802 in der Anmeldung erwähnt
- BOERSMA, ANTONIUS W.M. ET AL: "Bax upregulation is an early event in cisplatin-induced apoptosis in huma testicular germ-cell tumor cell line NT2, as quantitated by flow cytometr" CYTOMETRY (1997), 27(3), 275-282, XP002118803 in der Anmeldung erwähnt

## Beschreibung

Die Erfindung betrifft ein Verfahren zur Bestimmung der Chemosensitivität von Zellen gegen mindestens eine Substanz durch Messung der durch die mindestens eine Substanz ausgelösten Apoptose.

### Chemosensitivitätstests

Die Behandlungs- und Heilungserfolge von Tumorerkrankungen haben seit der Einführung der Chemotherapie stark zugenommen. Beispielsweise lag die Überlebensrate bei kindlicher akuter lymphatischer Leukämie (ALL) Mitte der 60er Jahre bei weniger als 10%. Heutzutage liegt die Heilungschance bei über 70%. Die Medikamente, sog. Zytostatika, werden nach bestimmten Therapieplänen allein oder in Kombination mit anderen Wirkstoffen verabreicht. Mittlerweile gibt es eine unüberschaubare Anzahl verschiedener Therapiepläne, die empirisch ermittelt und ständig weiterentwickelt werden. Das Hauptkriterium für einen verbesserten Therapieplan ist eine bessere Überlebensrate (clinical outcome). Dieses Kriterium trifft für die Mehrheit der Patienten zu. Jedes Individuum besitzt aber eine unterschiedliche Ausstattung an Zellen mit unterschiedlichen Eigenschaften. Dies trifft insbesondere auf die Eigenschaften von Tumorzellen zu. In einigen Studien konnte gezeigt werden, dass die Therapie für eine Subpopulation extrem gut wirkt, während sie für andere Patienten aufgrund von medikamentresistenten Tumoren wenig oder überhaupt nicht wirksam ist. (Lacombe et al., Blood, 84, 716-723 (1994), Smit et al. International Journal of Cancer 51, 72-78 (1992)). Dabei konnte gezeigt werden, daß nicht nur die Wirksamkeit verschiedener Medikamente, sondern auch die wirksame Dosis eines Medikamentes individuell unterschiedlich sein kann. Die Ermittlung der individuellen Dosierung eines Medikamentes ist für den Patienten von Bedeutung, damit er einerseits soviel an Medikamenten bekommt, wie für seine Heilung notwendig ist, und damit andererseits die toxischen Auswirkungen der Therapie und das Risiko einer durch die Chemotherapie induzierten sekundären Krebserkrankung so gering wie möglich gehalten werden. So gut die aktuell gebräuchlichen Therapiepläne auch sind, die Fortschritte der letzten Jahre stagnieren hinsichtlich der mittleren Überlebensrate. Eine weitere deutliche Verbesserung der Chemotherapie sollte durch die Erstellung individuell angepasster Therapiepläne möglich sein.

Um diese Problematik anzugehen haben einige Forscher versucht, die individuelle Sensitivität von Patiententumoren gegenüber Zytostatika *in vitro* zu messen. Die meisten Chemosensitivitätstests, die gegenwärtig verwendet werden, basieren wenigstens teilweise auf dem von Salmon (Salmon et al. Science, 197, 461-463 (1977)) entwickelten Agar-Tumorkultur-Test. Diese Tests messen die Zellproliferation.

Ein zweiter Typ von Chemosensitivitätstests umfasst den Ausschluss von (Fluoreszenz)-Farbstoffen oder die Freisetzung des radioaktiven Chromisotops ⁵¹Cr, mit dem die Zerstörung der Zellmembran durch direkte oder indirekte Zytostatikawirkung gemessen wird. Eine Modifikation des früheren Farbstoffausschluß-Tests ist der sogannte DiSC-Assay (Weisenthal, Kern Oncology 5: 92-103 (1991)), der durch einen zweiten Färbevorgang zusätzlich zwischen normalen und Tumorzellen differenziert.

Der dritte Typ von Chemosensitivitätstests bestimmt Parameter des zellulären Metabolismus als Maß für die Schädigung durch Zytostatika. Dieser Testtyp umfasst den radiometrischen BACTEC-Test (von Hoff D., Forseth B., Warfel L., in Salmon Trent(Eds.) Human tumor cloning, pp. 656-657, Grune & Stratton, Orlando (1984)) , den MTT-Test (Freund A. et al. European Journal of Cancer 34:895-901 (1998), Kaspers G.J.L. Blood 92:259-266 (1998), Pieters R. et al. Leukemia 12, 1344-1348 (1998), Klumper E. et al. British Journal of Haematology 93:903-910 (1996), Hwang W.S. et al. Leukemia Research 17:685-688 (1993)) und seinen Variationen, den ATP-Test (Kangas L., Gronroos M., Nieminen A., Medical Biology 62: 338-343 (1984)) und den sogenannten FCA-Test (Meitner P., Oncology 5: 75-81, (1991), Rotman B, Teplitz C., Dickinson K., Cozzolino J., Cellular and Developmental Biology 24: f1137-1146 (1988)).

Agar-Kultur-Assays haben den großen Nachteil, dass bei weitem nicht alle Tumorzellen in den Agar-Kulturen wachsen. Dies gilt insbesondere für lymphatische Leukämien und Lymphome (Veerman A.J.P., Pieters R. British Journal of Haematology 74:381-384 (1990)). Beim bekanntesten Vertreter dieses Assay-Typs, dem Clonogenen Assay, liegt der Prozentsatz von auswertbaren Zellpopulationen nur bei 30-40%. Die Zellen müssen sehr lange (10-20 Tage) wachsen, bevor die Auswertung erfolgt. Außerdem ist der Arbeitsaufwand immens.

Die Farbstoff-Ausschluss-Tests wie auch der ⁵¹Cr-Freisetzungstest bestimmen den Anteil von Zellen mit defekter Zellmembran. Diese Tests beinhalten häufig eine Fixierung und anschließende mikroskopische Auswertung der Zellen bzw. die Messung der Radioaktivität im Überstand. Solche Tests sind aufgrund der Messung eines recht groben Parameters, der Zellmembranintegrität, unspezifisch und können nicht zwischen einer spezifischen Anti-Tumor-Wirkung einer Substanz oder einer physikalischen oder z.B. durch eine starke Verätzung oder Oxidation verursachten Zellschädigung durch eine Substanz unterscheiden. Tests dieses Typs sind folglich auch bei Substanzen positiv, die generell alle Zellen schädigen und damit nicht als Antitumor-Medikament geeignet sind. Der wohl aussagekräftigste Test des Farbstoff-Ausschluß-Typs ist der DiSC-Test. Durch zweifache Anfärbung kann er im Gegensatz zu anderen Farbstoff-Ausschlusstests zwischen der Zellschädigung von Tumorzellen und normalen Zellen unterscheiden. Die Zweifach-Färbung und die anschließende mikroskopische Auswertung sind aber extrem zeitaufwendig. Außerdem variieren die Ergebnisse abhängig von der Person, die die Auswertung durchführt, und dem Bildausschnitt, der unter dem Mikroskop ausgewertet wird.

Für den radiometrische Tests wie z.B. den ⁵¹Cr-Freisetzungstest gelten ganz generell: Der Einsatz von radioaktiven Isotopen in diagnostischen Tests wird heutzutage wegen des Gefährdungspotentials und der Entsorgungsproblematik zunehmend vermieden. Hinzu kommt, dass inzwischen Fluoreszenz- und Luminiszenztechniken gleiche Sensitivitäten erreichen können bei häufig kürzerer Gesamttestdauer.

Von den Chemosensitivitäts-Tests, die den Metabolismus einer Zelle als Maß für die Proliferation verwenden, wird am häufigsten der MTT-Test und seine Variationen verwendet. Der MTT-Test hat nach einer 4-tägigen Inkubation der Zellen mit diversen Zytostatika eine relativ kurze Testdauer von etwa 4 Stunden. Außerdem können 96 Proben parallel in einer Mikrotiterplatte mittels eines ELISA-Auslesegerätes ausgewertet werden. Dadurch wird ein passabler Durchsatz bei relativ geringem Personalaufwand gewährleistet. Der MTT-Test basiert auf der Umsetzung von 3-[4,5-Dimethylthiazol-2-yl]-2,5-diphenyltetrazoliumbromid in ein blaues Formazan-Produkt. Die Umsetzung wird durch Dehydrogenasen katalysiert, die nur in lebenden Zellen aktiv sind. Der MTT-Test ist abhängig von einer konstitutiven Basisaktivität der untersuchten Zellen. Es hat sich aber herausgestellt, dass einige Zelltypen, z.B. besonders häufig einige FAB-Subtypen von akuter myeloischer Leukämie, eine stark verringerte Dehydrogenaseaktivität besitzen (Santini V. et al. Hematological Oncology 7:287-293 (1989)). Die Chemosensitivität dieser Tumorzellen kann daher nicht mit einem MTT-Test bewertet werden. Ein weiteres Problem sind die entstehenden wasserunlöslichen Formazankristalle. Diese müssen durch Zusatz von organischen Lösungsmitteln z.B. Dimethylsulfoxid oder Isopropanol in Lösung gebracht werden. Die Erfahrung hat gezeigt, dass dadurch Probleme bei der Reproduzierbarkeit des Tests auftreten.

### Apoptose

Durch Arbeiten diverser Forschergruppen (z.B. Sen, S. et al. FEBS Lett. 307:122-127, Darzynkiewicz et al. Cytometry 13: 795-808 (1992), Fulda S., Los M., Friesen C., Debatin K.M. Int. J. Cancer 76:105-114 (1998)) erscheint es wahrscheinlich, dass durch Zytostatika verursachter Zelltod generell über den Mechanismus der Apoptose verläuft. Die Apoptose stellt einen von der Zelle selbst programmierten Zelltod dar, der durch physiologische Faktoren im Organismus induziert werden kann oder durch Chemikalien wie Zytostatika ausgelöst werden kann. Der programmierte Zelltod spielt eine außerordentlich große Rolle bei der Zytostase z.B. des Immunsystems. So werden beispielsweise T-Zellen, die gegen körpereigene Strukturen gerichtet sind, durch die Apoptose aus dem Organismus entfernt. Ansonsten kann es zu Symptomen einer Autoimmunerkrankung kommen, z.B. Lupus Erythematodes, arthritische Erkrankungen oder der Erkrankung durch einen T-Zell Tumor. Substanzen wie der von Makrophagen synthetisierte Tumornekrosefaktor alpha sind natürliche Induktoren der Apoptose.

Apoptose verläuft nach einem programmierten, einheitlichen Schema, bei dem in einer sehr frühen Phase Phosphatidylserin, das sich normalerweise ausschließlich auf der Innenseite der Zellmembran befindet, auf der Außenseite präsentiert wird (Inversion). Dieses Ereignis tritt deutlich früher auf, als die Stunden später erfolgende Fragmentierung der DNA und Auflösung der Zellmembran in der späten Phase der Apoptose.

Neben dem Zelltod durch Apoptose existiert eine andere Form von Zelltod, die z.B. physikalisch, durch osmotischen Schock, Verätzung oder Oxidation ausgelöste Nekrose. Diese äußert sich durch stark degenerative Schäden an der Zelle.

Medenica (US 5736129) bestimmt das Ausmaß der Zellschädigung durch Zytostatika, indem er die Zellen mit Propidiumiodid anfärbt und anschließend die angefärbten Zellen im FACS (fluorescence activated cell sorter) auszählt. Dieser Ansatz hat aber den großen Nachteil, nicht genau zwischen Apoptose und Nekrose zu unterscheiden, und kann demzufolge den spezifischen Wirkmechanismus von Zytostatika nicht erfassen. Außerdem ist der Zellbedarf für eine aussagekräftige Analyse sehr hoch.

Die Erkennung von auf der Zelloberfläche präsentiertem Phosphatidylserin ist zur spezifischen Detektion von Apoptose bestens geeignet, da die Inversion von Phosphatidylserin ein sehr früher Schritt der Apoptose ist und die Anfärbung sowohl an fixierten, als auch an suspendierten Zellen schnell und leicht durchführbar ist. In bisher beschriebenen Tests wird als Phosphatidylserin bindender Marker stets Annexin V verwendet. Dieses zeichnet sich durch einen sehr niedrigen Kd von 5×10⁻¹⁰ M aus. Nachteilig ist die starke Kalzium-Kationen-Abhängigkeit der Bindung. Diese Tests bestehen alle aus einer Inkubationsphase ohne oder mit Substanzen (z.B. Zytostatika), Sedimentation und Waschen in PBS und anschließender Resuspension in Kalzium-haltigen Färbepuffer mit markiertem Annexin V. Die angefärbten Zellen können anschließend im FACS oder unter dem Fluoreszenzmikroskop gezählt werden. Da alle bisherigen Testprotokolle Waschschritte enthalten und daher zu den heterogenen Assays zählen, sind diese Tests relativ arbeitsaufwendig und fehleranfällig. Diese Tests sind alle dadurch gekennzeichnet, dass die Apoptose zu einem bestimmten Zeitpunkt nach Induktion durch Substanzen gemessen wird, was einer Endpunktmessung entspricht. Da die Apoptose ein dynamischer Prozess ist, der ein Maximum durchläuft, das sowohl von der untersuchten Substanz, als auch vom Zelltyp abhängt, und dann in einer sekundären Nekrose ausläuft, ist a priori nicht bekannt, zu welchem Zeitpunkt das Ausmaß der Apoptose gemessen werden muss. Daraus folgt, dass im Prinzip für jeden Patienten mehrere Proben mit z.B. Tumorzellen nach unterschiedlichen Inkubationszeiten hinsichtlich der Chemosensitivität bestimmt werden müssen.

Diese Verfahrensweise ergibt sich auch aus einem Katalog der Firma Clontech aus dem Jahr 1998/99, Seite 71 bis 74 in Verbindung mit dem authentischen Durchführungsprotokoll APO Alert ®Annexin V.

TOH, H.C. et.al. beschreiben in Leukemia and Lymphoma, (1998) 31 (1-2) S. 195 - 208 eine Methode zum Nachweis der Apoptose durch Bindung von Annexin V an Lymphoma- und T-Cell-Leukemiezellen. Die Apoptose wird durch Vinorelbine und Vincristine ausgelöst. Dabei wird auch eine Endpunktmessung durchgeführt.

Kravtsow, V.D. et.al. beschreiben in Blood (1998), 92 (3), S. 968- 980 die Bestimmung einer Apoptosekurve durch zeitabhängige Messung der optischen Dichte einer Probe. Dabei wird kein Marker verwendet. Diese Methode erfasst einen Parameter, der indirekt mit Apoptose assoziiert ist und darüber hinaus vergleichsweise unspezifisch und störungsanfällig ist.

Boersma, A. W.M. et. al. beschreiben in Cytometry (1997), 27 (3), 275 - 282 ebenfalls eine Endpunktmessung, bei der zu verschiedenen Zeiten Apoptose Induktoren enthaltende Inkubationen abgebrochen werden und das Ausmaß der Apoptose in den jeweiligen Ansätzen bestimmt wird.

Die bisher beschriebenen Phosphatidylserin-Tests ermöglichten keine unmittelbare Messung des zeitlichen Verlaufs der Apoptose. Mit den bisherigen Testvorschriften mußte für eine bestimmte Kombination aus einem Zelltyp und einer Konzentration einer Substanz eine Serie von Experimenten durchgeführt werden, um den zeitlichen Verlauf der Apoptose zu erfassen. Der Zeit- und Personalaufwand, wie auch der Zellmaterialbedarf für einen derartigen Test wäre derart hoch, dass eine hochparallele Testung von wenig Zellmaterial auf Chemosensitivität gegenüber verschiedenen Konzentrationen von vielen Substanzen unmöglich war.

Aufgabe der vorliegenden Erfindung war es, die genannten Nachteile des Standes der Technik zu überwinden.

Die Aufgabe wird gelöst durch ein Verfahren zur Bestimmung der Chemosensitivität von Zellen gegen mindestens eine Substanz durch Messung der durch die mindestens eine Substanz ausgelösten Apoptose, wobei die Zellen im wesentlichen gleichzeitig mit mindestens einem Marker, dessen spezifische Bindefähigkeit an Phosphatidylserin detektierbar ist und mit der mindestens einen Substanz inkubiert werden und die Bindung zwischen Marker und Phosphatidylserin zeitlich aufgelöst detektiert wird.

Die hinsichtlich ihrer Chemosensitivität zu untersuchenden Zellen werden in Gegenwart der Substanzen, d.h. gleichzeitig oder mit vorgelegten Markermolekülen, inkubiert. Dadurch wird aus den bisher eingesetzten heterogenen Testprotokollen ein homogener Test "Mix and Measure", der die unmittelbare Messung der Kinetik des apoptotischen Vorganges ermöglicht. Wenn Annexin V als Marker eingesetzt wird, muss sichergestellt werden, dass die Kalziumionenkonzentration während der gesamten Meßzeit konstant und in der optimalen Konzentration bleibt. Dies kann z.B. dadurch erreicht werden, indem die Kalziumionenkonzentration abgepuffert wird. Die Kalziumionen-Abhängigkeit kann gänzlich umgangen werden, wenn anstelle von Annexin V als Marker andere Annexine, Annexinderivate, Annexinmuteine, Antikörper, Fab-Fragmente, single-chain Antikörper, Aptamere und/oder sonstige Proteine mit Bindungsstellen für Phosphatidylserin verwendet werden.

Um den Materialverbrauch weiter zu senken, ist eine Miniaturisierung möglich. Es konnte gezeigt werden, dass Zellen in weniger als 10 µl Medium einige Tage in Kultur gehalten werden können. Durch die Senkung der Zellzahl pro Probenkammer auf ≤ 1000 pro Test werden hochparallele Analysen von Proben mit äußerst wenig Patientenzellen, wie z.B. aus Feinnadelbiopsien, ermöglicht. Der geringe Zellbedarf ermöglicht auch die Hochdurchsatztestung (High Throughput Screening /HTS) von unbekannten Substanzen auf anti-Tumorwirkung.

Wenn die Apoptose von Zellen pathologischer Gewebe über die Phophatidylserininversion getestet wird, ist es von Vorteil, als Referenz gesunde Zellen des Gewebes sowie dieselben Zellen ohne Substanzzusatz mitzutesten. Insbesondere ist es sinnvoll als Kontrolle eine permanente Zell-Linie, deren Chemosensitivität bekannt ist, mitzutesten, um die Funktionalität des Tests zu dokumentieren und Fehler bei der Testdurchführung zu erkennen.

Als Substanzen kommen z.B. folgende Substanzen in Frage:
A) Zytostatika
   Abrin, Amethopterin, Acivin, Aclacinomycin A, Alanin-Mustard, Altretamin, Aminoglutethimid, Aminopterin, Amsacrin (mAMSA), div. anabolische Steroide, Anthrapyrazol, L-Asparaginase, 5-Axacytidin, Bazillus Calmette-Guerin, Bisantren, Buserelin, Busulfan, Butyryloxyethylglyoxal-dithiosemicarbazon, Camptothecin, Carbamat-ester, Carzinophyllin, CCNU, Chlorambucil, Chlorethylmethylcyclohexyl-nitrosoharnstoff, Chlorethylcyclohexylnitrosohamstoff, Chlorodeoxyadenosin, Corticotropin, Cyproteronacetat, Chlorotrianisen, Chlorozotozin, Chromomycin-A, Cytosinarabinosid (Ara-C), BCNU, Bleomycin, Cis-Platin, Carbo-Platin, Cladribine, Cyclophosphamid, Dactinomycin, Daunomycin, Daunorubicin, Decarbacin, Doxorubicin, DTIC, Dehydroemitin, 4-Demethoxydaunorubicin, Demothydoxorubicin, Deoxydoxorubicin, Dexamethason, Dibromodulcitol, Dichloromethotrexat, Diethylstilbestrol, Bis-(2-chloropropyl)- DL-Serin, Doxifluridin, Elliptiniumacetat, 4'-Epidoxorubicin, Epirubicin, Epoetin alpha, Erythropoeitin, Esorubicin, Estradiol, Etoposid, Fluoxymesteron, Flutamid, Folsäure, Fotemustin, Ftorafur, 4-FU, Fludarabine-Phosphat, 5-FU, Floxuridin, Galactitol, Galliumnitrat, Goserelin, G-CSF, GM-CSF, Hydrea, Hexamethylmelamin (HMM), Hydrocortison, Hydroxyprogesteron, 4-Hydroperoxycyciophosphamid, ICRf 159, Idamycin, Ifosfamid, Immunglobulin IGIV, Interferon, Kobalt-Proporphyrinkomplex, Leucovorin Calcium, Leuprolid, Levadopa, Levothyroxin, Lindan, Liothyronin, Liotrix, Lomustin, Levamisole, Masoprocol, Maytansin, Menogaril, 6-Mercaptopurin, Methosalen, Methylesteron, Methyl-lomustin, Mithracin, Mithramycin, Mitotan, Mitoxantron, Methotrexat (MTX), 6 MP, Mechlorethamin-Hydrochlorid, Medroxyprogesteron, Megestrolacetat, Melphalan, Mesna, Mitomycin-C, Nandrolon, Natriumphosphat P32, Navelbin, Neocarzinostatin, Nitrofururazon, nHuIFNa, nHuIFNb, nHuIFNp, Octreotidacetat, Ondansetrone Hydrochlorid, Dinatrium-Pamidronat, Pentamethylmelamin (PMM), Pentostatin, Peptiochemio, Plicamycin, Prednimustin, Probroman, Procarbazin, Profiromycin, Paraplatin, Prednisolon, Prednison, RazoxanrIFNa-2a, Rubidazon, rIFNa-2b, rIFNb-1b, rIFNt-1b, rIL-2, rTNF, Semustin, SPG 827 (Podophyllinderivat), Spirogermanium, Streptonigrin, Somatostatin, Streptozocin, Tamoxifen, Taxol, Thio-TEPA, 6-Thioguanin, Tenoposid, Testolacton, Testosteron, 3-TGDR, rTNF, Thyroglobulin, Thyrotropin, Trilostan, Uracil-Mustard, VP-16, Vincristin (VCR), Vinblastin (VBL), Verapamil, Vindesin, Vinzelidin, Vitamin A-Säure, Vitamin A-Analoga, Zinostatin.
B) Peptide und Peptoide
C) Nukleinsäuren und -Derivate
D) Peptidnukleinsäuren (PNA's)
E) Hybride aus RNA's, DNA's, PNA's u. Derivate

Zur Erkennung von extrazellulärem Phosphatidylserin müssen Zellen in Gegenwart eines Markers mit den zu untersuchenden Substanzen inkubiert werden. Diese Marker können Antikörper, Fab-Fragmente, single-chain Antikörper, Aptamere und/oder sonstige Proteine mit Bindungsstellen für Phosphatidylserin wie Annexine eingesetzt werden. Diese Marker können einen Farbstoffanteil, kolloidales Edelmetall, ein radioaktives Isotop und/oder Seltenerdenmetallchelate aufweisen.

Die Detektion erfolgt bevorzugt mittels bildgebender Verfahren, wie Fluoreszenznachweisverfahren, insbesondere auf Basis von konfokaler oder konventioneller Mikroskopie. Die intakten fluoreszenzmarkierten Phosphatidylserin-präsentierenden Zellen werden z.B. mit einer CCD-Kamera aufgenommen und anschließend mit Hilfe eines Bildverarbeitungsprogramms gezählt. Zellen, die sich durch einen DNA-Farbstoff, wie z.B. Propidiumiodid anfärben lassen, werden als nekrotisch angesehen und von der Zahl der über Phosphatidylserin fluoreszenzmarkierten Zellen abgezogen, so daß die Zahl der apoptotischen Zellen ermittelt wird. Diese wird dann in Bezug zur Gesamtzellmenge gesetzt, die durch automatische Bildauswertung im Phasenkontrast-Mikroskop ermittelt wird. Geeignet wäre aber auch Fluoreszenzkorrelationsspektroskopie (FCS) und konfokale Fluorimetrie, wobei z.B. die Konzentration der fluoreszenten Markermoleküle im Überstand und/oder auf der Zelloberfläche gemessen wird. Bei Mehrfachanfärbungen z.B. durch einen zweiten Marker oder denselben Marker mit einem anderem Fluoreszenzfarbstoff können im Überstand wie auch auf der Zelloberfläche Kreuzkorrelations- oder Zweifarbenkoinzidenzfluorimetrische Messungen durchgeführt werden. Dabei werden bei beiden Verfahren simultan zwei verschiedene Fluoreszenzfarbstoffe detektiert. Bei der Kreuzkorrelation werden die zeitabhängigen Fluoreszenzfluktuationssignale der beiden Messkanäle kreuzkorreliert. Bei der Zweifarbenkoinzidenzfluorimetrie gibt es genau dann ein positives Signal, wenn am selben Ort zeitgleich beide Fluoreszenzsignale auftreten. Durch solche Zweifarbenanalysen kann die Spezifität des Nachweises für die Phosphatidylserininvertierung noch gesteigert werden. Von besonderem Nutzen ist die Anwendung von FIDA und PIDA wie in der WO 98/16814 und PCT/EP 98/06165 beschrieben. Dadurch kann die mehrfache Bindung von fluoreszenten Markermolekülen an apoptotische Zellen aufgrund der gesteigerten Fluoreszenzemission leicht gemessen werden. Durch den Einsatz von 2D-FIDA kann die Spezifität noch weiter gesteigert werden. Als sehr leistungsstark hat sich eine Kombination der erwähnten Fluoreszenzmesstechniken mit einer relativen Bewegung von Proben- und Messvolumen gegeneinander gezeigt. Da auf diese Weise sehr viel mehr Zellen pro Zeiteinheit beobachtet werden können, verbessert sich die Signalstatistik und damit das Signal/Rausch-Verhältnis drastisch. Insbesondere ist dann auch eine Quantifizierung der Bindung von fluoreszenten Markermolekülen an Zellen möglich.

Durch die Markierung von Phosphatidylserin werden apoptotische Zellen, aber auch Zellen mit defekten Zellmembranen angefärbt, da dann auch die Phosphatidylserine auf der Innenseite der Zellmembranen zugänglich werden. Wenn man den zeitlichen Verlauf der Apoptose verfolgt, so werden zunächst vermehrt intakte, aptoptotische Zellen äußerlich angefärbt. Es schnüren sich kleinere ebenfalls angefärbte apoptische Vesikel ab (apoptotic bodies). In der Endphase gehen apoptotische Zellen in die sekundäre Nekrose über und zerfallen. Nekrotische Zellen sind dadurch gekennzeichnet, daß die Zellmembranen in Fragmente zerfallen. Apoptose muss von Nekrose unterschieden werden, damit die Wirkung von wirksamen Zytostatika, die alle über einen Apoptosemechanismus verlaufen, spezifisch detektiert wird. Um zwischen apoptotischen und nekrotischen Zellen zu unterscheiden, können zusätzlich Marker zum Ausschluss von Nekrosen, zusammen mit dem phosphatidylserin bindendem-Marker eingesetzt werden, beispielsweise Farbstoffe, die mit Nukleinsäuren in Wechselwirkung treten, aber intakte Zellmembranen nicht durchdringen können (z.B. Propidiumiodid , BOBO™ (Molecular Probes)).

Der zeitliche Verlauf der Apoptose kann so mittels der Markierung von Phosphatidylserin "Online" verfolgt werden.

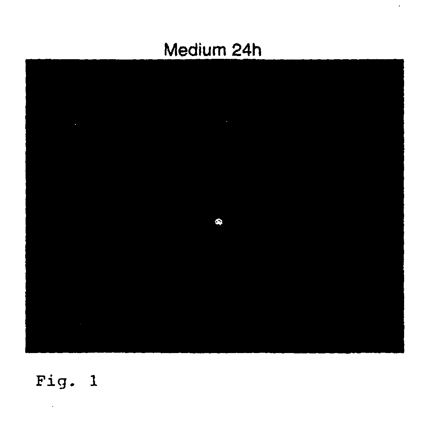

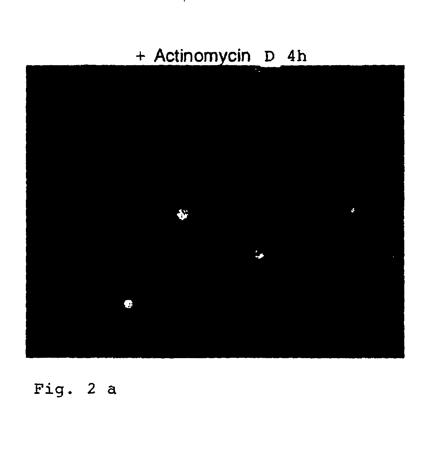

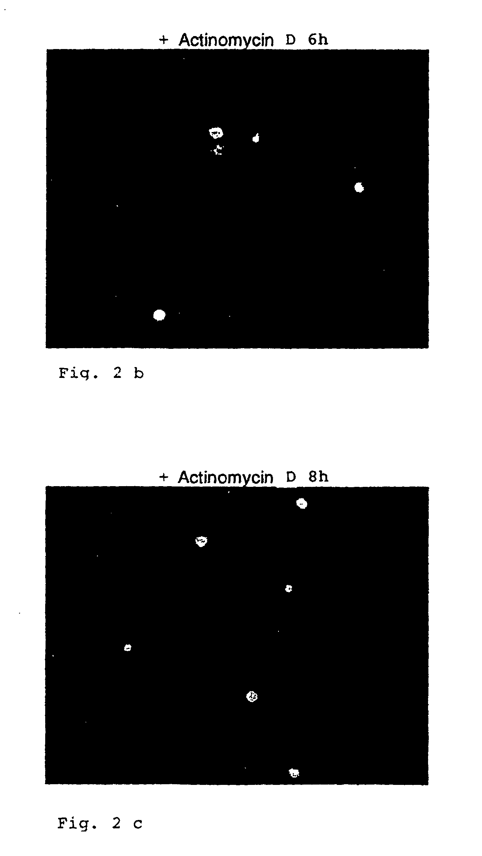

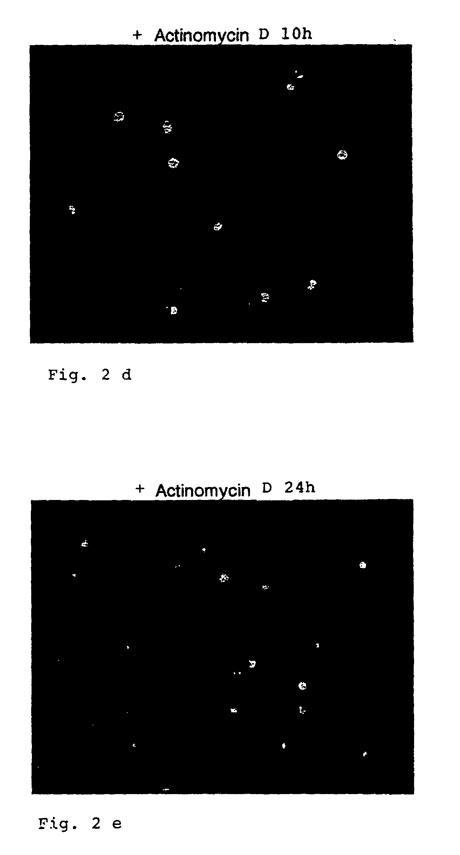

Die Figuren 1 und 2 zeigen fluoreszenzmikroskopische Aufnahmen apoptotischer Zellen (siehe Beispiel 2).

Es ist von Bedeutung, das Ausmaß der Apoptose auf die Gesamtzahl der untersuchten Zellen zu normieren. Dies kann z.B. durch Messung der Lichtabsorption, Streulicht, Leitfähigkeitsmessung oder mikroskopische Auswertung geschehen. Die Normierung ist notwendig, um das Ausmaß der Zellschädigung durch verschiedene Zytostatika vergleichen zu können.

### Beispiel:

Ein stark wirkendes Zytostatikum, das das Wachstum der Zellen unmittelbar stoppt und alle Zellen in Apoptose überführt, kann aufgrund der geringen Zellzahl deutlich weniger Apoptose aufweisen, als im Falle eines schwächer wirkenden Zytostatikums, bei dem sich die Zellen zunächst noch vermehren können, um dann erst später zu einem geringen Teil in Apoptose zu gehen. Erst die Normierung berücksichtigt die Apoptose in Bezug auf die Gesamtzellzahl und bewertet das stärker wirkende Zytostatikum richtig.

Die Chemosensitivitätstestung von Zellen lässt sich besonders vorteilhaft mit einem Kit durchführen. Dieser Kit beinhaltet einen Probenträger mit mehreren Probenkammern, wobei eine Probenkammer mindestens eine Substanz und einen Marker zur Phosphatidylserindetektion und gegebenenfalls einen Marker zum Ausschluss von Nekrose enthält. Bei dem Probenträger kann es sich z.B. um eine handelsübliche Mikrotiterplatte handeln. Die zu testenden Substanzen und der Marker können dabei entweder als Trockensubstanz, in Lösung oder in Gegenwart von Matrixsubstanzen wie z.B. Salzen, Puffer, Kohlenhydraten, Carbonsäuren, Pyrimidinen, anorganischen oder organischen Nanopartikeln bis 1 µm Durchmesser vorliegen.

### Beispiel 1

**Material für die Durchführung des Chemosensitivitätstests:**
- Sterilbank (Holten, Antares)
- Fluoreszenzmikroskop (Carl Zeiss Jena)
- CO₂-Inkubator (WTC Binder)
- Vortex Mixer (Bender + Hobein AG)
- Kühlschrank (2-10°C) (Bosch)
- Gefrierschrank (-20°C) (Liebherr)
- Autoklav (H+P Labortechnik GmbH)
- Zentrifuge (Eppendorf)
- Pipetten (Eppendorf)
- Pipettenspitzen (ratiolab)
- Mikrotiterplatten (96 Kammern) (Falcon)
- 12,50 ml Röhrchen (Falcon, Greiner)
- RPMI 1640 Medium (Gibco)
- Patientenblut oder -knochenmark
- Annexin V-Alexa 568™ (Boehringer Mannheim)
- BOBO™ (Molecular Probes)
- Dulbecco's PBS (Gibco)

### Testdurchführung:

### Zellzahlbestimmung

10 µl einer Zellsuspension werden in eine Neubauerkammer eingefüllt. Unter einem Lichtmikroskop werden die Zellen in den 16 Kleinstquadraten gezählt. Durch Multiplikation der Zellzahl mit 10000 erhält man die Zellzahl pro ml.

### Präparationen einiger Zytostatika-Lösungen

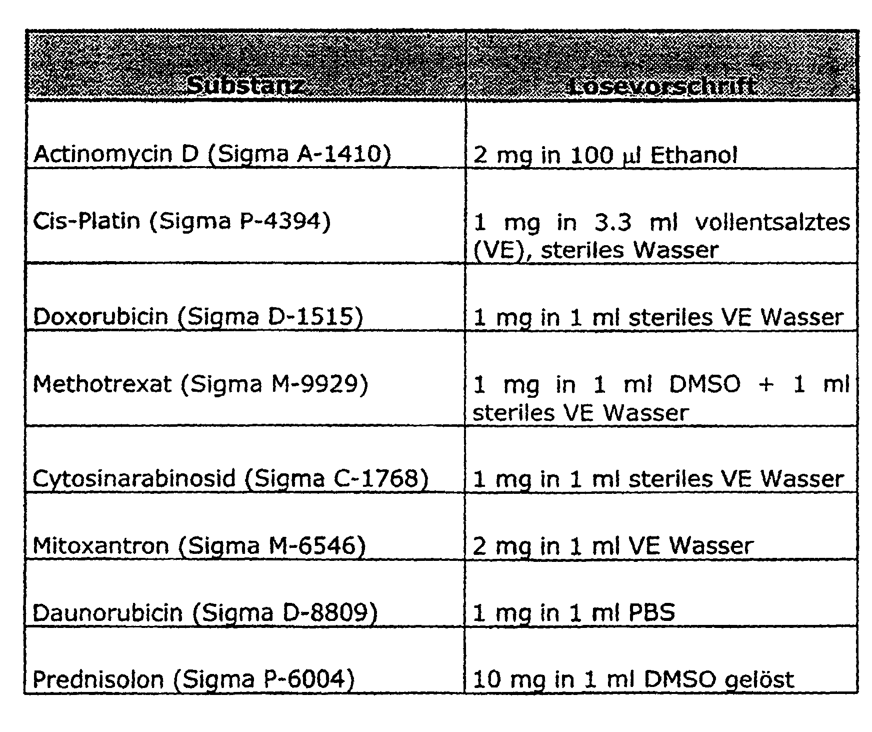

### Mischen und Inkubation mit Zytostatika

Die Zellen werden gemeinsam mit 0.5 µg/ml BOBO™ und 50 µM Annexin V-Alexa 568™ in Gegenwart einer konstant 3 mM Kalziumionenkonzentration mit den Zytostatika inkubiert.

### Quantifizierung im Fluoreszenzmikroskop

Im Stundentakt werden pro Probe mit dem in einer CCD-Kamera und Bildauswertungssoftware ausgestatteten Fluoreszenzmikroskop zunächst die durch Annexin V rot fluoreszenzmarkierten Zellen, dann durch die durch BOBO grün markierten Zellen und abschließend die Gesamtheit der Zellen in Phasenkonstrast-Modus gezählt. Die durch BOBO angefärbten nekrotischen Zellen werden von der Zahl der Annexin V-positiven Zellen abgezogen und in Bezug zur Gesamtzahl der Zellen gesetzt. Dadurch erhält man den normierten Prozentsatz apoptotischer Zellen. Mit zunehmender Inkubationsdauer steigt der Anteil apoptotischer Zellen zunächst, um nach einigen Stunden wieder abzunehmen, wenn mehr und mehr Zellen in die Endphase der Apoptose, der sekundären Nekrose, übergehen. Das maximale Ausmaß der Apoptose ist ein Maß für die Wirksamkeit eines Zytostatikum.

### Beispiel 2

100000 Jurkat P40 Zellen wurden in Gegenwart von 2 mm CaCl₂ und fluoreszenzmarkiertem Annexin V mit 1 µg/ml Actinomycin D zeitgleich gemischt und über einen Zeitraum von bis zu 24 h bei 37° C in 5 % CO₂ inkubiert. Nach 4, 6, 8, 10 und 24 h wurde je ein Aliquot entnommen, auf einem Objektträger aufgebracht, fixiert und fluoreszenzmikroskopisch analysiert. Die Mediumkontrolle zeigt selbst nach 24 h mit der Anfärbung von nur 1 Zelle eine sehr geringe Spontanapoptoserate. Mit zunehmender Inkubationszeit nimmt der Grad der Apoptose - erkennbar an der zunehmenden Zahl gefärbter Zellen im Beobachtungsfeld - zu. Nach 24 h werden schon kleinere Zellfragmente sichtbar, die auf sekundäre Nekrose hinweisen. Bei noch längerer Wartezeit und fortschreitender Zellzerstörung wären irgendwann keine fluoreszenten Zelltrümmer mehr sichtbar. Es wurde gezeigt, dass die Apoptose in einer Lösung durch Bildaufnahme nach verschiedenen Zeitpunkten zeitlich aufgelöst verfolgt werden kann. Dabei wurde die Reaktionsmischung zu Beginn hergestellt und anschließend nur noch die zunehmende Apoptose mittels bildgebenden Verfahren analysiert.

Figur 1 zeigt die entsprechende Aufnahme für die Kontrolle mit Medium nach 24 h. Die Figuren 2 a - 2 e zeigen den Verlauf nach Zugabe von Actinomycin.

## Patentansprüche

1. Verfahren zur Bestimmung der Chemosensitivität von Zellen gegen mindestens eine Substanz durch Messung der durch die mindestens eine Substanz ausgelösten Apoptose unter Vermeidung von Reihentests, wobei die Zellen im wesentlichen gleichzeitig mit mindestens einem Marker, dessen spezifische Bindefähigkeit an Phosphatidylserin detektierbar ist und mit der mindestens einen Substanz inkubiert werden und die Bindung zwischen Marker und Phosphatidylserin zeitlich aufgelöst detektiert wird.

2. Verfahren nach Anspruch 1, **dadurch gekennzeichnet, dass** der mindestens eine Marker vor und/oder während der Inkubation der Zellen mit der mindestens einen Substanz zugesetzt wird.

3. Verfahren nach Anspruch 1 und/oder 2, **dadurch gekennzeichnet, dass** die Zellen tierische oder humane Zellen sind, insbesondere Leukämiezellen, Zellen solider Tumore, Zellen pathologischer Organe und/oder Referenzzellen wie beispielsweise Zellen aus anderen als den pathologischen Organen oder Zellen aus gesunden Bereichen pathologischer Organe.

4. Verfahren nach einem der Ansprüche 1 bis 3, **dadurch gekennzeichnet, daß** eine Referenzmessung ohne Zusatz der mindestens einen Substanz durchgeführt wird.

5. Verfahren nach einem der Ansprüche 1 bis 4, **dadurch gekennzeichnet, dass** als Substanzen pharmazeutische Wirksubstanzen, Chemotherapeutika, Umweltschadstoffe, Peptide, Nukleinsäuren oder Derivate hiervon, PNAs und/oder Nukleinsäurehybride eingesetzt werden.

6. Verfahren nach einem der Ansprüche 1 bis 5, **dadurch gekennzeichnet, dass** als Marker Antikörper, Fab-Fragmente, single-chain Antikörper, Aptamere und/oder sonstige Proteine mit Bindungsstellen für Phosphatidylserin wie Annexine eingesetzt werden.

7. Verfahren nach einem der Ansprüche 1 bis 6, **dadurch gekennzeichnet, dass** der Marker einen Farbstoffanteil, ein kolloidales Edelmetall, ein radioaktives Isotop und/oder Seltenerdenmetallchelate aufweist.

8. Verfahren nach einem der Ansprüche 1 bis 7, **dadurch gekennzeichnet, dass** apoptotische Zellen von nekrotischen Zellen unterschieden werden, insbesondere durch Ko-Inkubation mit einem Marker für nekrotische Zellen, insbesondere mit einem mit Nukleinsäuren in Wechselwirkung tretenden Farbstoff, der intakte Zellmembranen nicht durchdringen kann.

9. Verfahren nach einem der Ansprüche 1 bis 8, **dadurch gekennzeichnet, dass** zur Detektion bildgebende Verfahren, wie Fluoreszenznachweisverfahren, insbesondere Verfahren auf Basis von konfokaler oder konventioneller Mikroskopie eingesetzt werden.

10. Verfahren nach einem der Ansprüche 1 bis 9, **dadurch gekennzeichnet, dass** die die Zahl der als apoptotisch Identifizierten Zellen auf die Gesamtzahl der Zellen normiert wird.

11. Verfahren nach einem der Ansprüche 1 bis 10, **dadurch gekennzeichnet, dass** die Detektion mit einer zeitlichen Auflösung von Stunden oder in größeren Zeltabständen erfolgt.

12. Verfahren nach einem der Ansprüche 1 bis 11, **dadurch gekennzeichnet, dass** als Marker Annexin V In Gegenwart von Calcium im Konzentrationsbereich von 0.1 bis 30 mM, bevorzugt 1 bis 10 mM, verwendet wird.

13. Verfahren nach einem der Ansprüche 1 bis 12 zur Suche nach apoptotisch wirkenden Substanzen.

14. Verwendung eines Kits enthaltend mindestens ein Zytostatikum und mindestens einen Marker, dessen spezifische Bindefähigkeit an Phosphatidylserin detektierbar ist, wobei diese entweder als Trockensubstanz, in Lösung oder in Gegenwart von Matrixsubstanzen vorliegen. In einem Verfahren nach einem der Ansprüche 1 bis 13 zur Bestimmung der Chemosensitivïtät von Zellen gegen mindestens eine Substanz.

## Claims

1. A method for determining the chemosensitivity of cells towards at least one substance by measuring the apoptosis induced by said at least one substance avoiding serial tests, wherein the cells are incubated essentially concurrently with at least one marker which has binding sites for phosphatidylserine and which can be detected, and with said at least one substance, and the binding between the marker and phosphatidylserine is detected in a time-resolved manner.

2. The method according to claim 1, **characterized in that** said at least one marker together with said at least one substance is added prior to and/or during the incubation of the cells.

3. The method according to claim 1 and/or 2, wherein said cells are animal, including human, cells, especially leukemia cells, cells of solid tumors, cells of pathological organs, and/or reference cells, such as cells from organs other than the pathological ones, or cells from healthy regions of pathological organs.

4. The method according to any of claims 1 to 3, **characterized in that** a reference measurement is performed without the addition of said at least one substance.

5. The method according to any of claims 1 to 4, **characterized in that** pharmaceutically active substances, chemotherapeutic agents, environmental pollutants, peptides, nucleic acids or derivatives thereof, PNAs and/or nucleic acid hybrids are employed as said substances.

6. The method according to any of claims 1 to 5, **characterized in that** antibodies, F_{ab} fragments, single-chain antibodies, aptamers and/or other proteins having binding sites for phosphatidylserine are employed as said markers.

7. The method according to any of claims 1 to 6, **characterized in that** said marker comprises a dye portion, a colloidal precious metal, a radioactive isotope, and/or rare-earth metal chelates.

8. The method according to any of claims 1 to 7, **characterized in that** apoptotic cells are distinguished from necrotic cells, especially by co-incubation with a marker for necrotic cells, especially with a dye interacting with nucleic acids which cannot permeate intact cell membranes.

9. The method according to any of claims 1 to 8, **characterized in that** imaging methods, such as fluorescence detection methods, especially methods based on confocal or conventional microscopy, are employed for detection.

10. The method according to any of claims 1 to 9, **characterized in that** the number of cells identified as apoptotic is standardized for the total number of the cells.

11. The method according to any of claims 1 to 10, **characterized in that** the detection is performed with a time resolution of hours or at greater time intervals.

12. The method according to any of claims 1 to 11, **characterized in that** annexin V is used as the marker in the presence of calcium in a concentration range of from 0.1 to 30 mM, preferably from 1 to 10 mM.

13. The method according to any of claims 1 to 12 used for the screening for apoptotically effective substances.

14. Use of a kit containing at least one cytostatic agent and at least one marker whose specific binding capacity to phosphatidylserine can be detected, said substances being present as dry substances, involution, or in the presence of matrix substances, in a method according to any of claims 1 to 13 for determining the chemosensitivity of cells towards at least one substance.

## Revendications

1. Procédé de détermination de la chimiosensibilité de cellules vis à vis d'au moins une substance par mesure de l'apoptose déclenchée par la au moins une substance, qui évite des tests en série, dans lequel on incube les cellules pratiquement simultanément avec au moins un marqueur qui comporte des sites de liaison pour la phosphatidylsérine et qui est détectable, et avec le au moins une substance, et on détecte ensuite la liaison entre le marqueur et la phospatidylsérine après un certain intervalle de temps.

2. Procédé selon la revendication 1, **caractérisé en ce qu'**on ajoute le au moins un marqueur avec le au moins une substance avant et/ou pendant l'incubation des cellules.

3. Procédé selon la revendication 1 et/ou 2, **caractérisé en ce que** les cellules sont des cellules animales ou humaines, notamment des cellules leucémiques, des cellules de tumeurs solides, des cellules d'organes pathologiques et/ou des cellules de référence comme par exemple des cellules provenant d'autres organes non pathologiques ou des cellules provenant de régions saines d'organes pathologiques.

4. Procédé selon une des revendications de 1 à 3, **caractérisé en ce qu'**on effectue une mesure de référence sans ajouter la au moins une substance.

5. Procédé selon une des revendications de 1 à 4, **caractérisé en ce qu'**on utilise en tant que substances des substances actives pharmaceutiques, des agents chimiothérapiques, des substances polluantes pour l'environnement, des peptides, des acides nucléiques ou des dérivés d'entre eux, des ANP ou des hybrides d'acide nucléique.

6. Procédé selon une des revendications de 1 à 5, **caractérisé en ce qu'**en tant que marqueur, on utilise des anticorps, des fragments Fab, des anticorps à une seule chaîne, des aptamères et/ou des protéines quelconques comportant des sites de liaison à la phosphatidylsérine telles que l'annexine.

7. Procédé selon une des revendications de 1 à 6, **caractérisé en ce que** le marqueur comporte une portion colorante, un métal noble colloïdal, un isotope radioactif, et/ou un chélate de métal de terre rare.

8. Procédé selon une des revendications de 1 à 7, **caractérisé en ce qu'**on différencie les cellules apoptotiques des cellules nécrotiques, notamment par co-incubation avec un marqueur pour les cellules nécrotiques, en particulier avec un colorant entrant en interaction avec les acides nucléiques qui ne peut pas pénétrer à l'intérieur de membranes cellulaires intactes.

9. Procédé selon une des revendications de 1 à 8, **caractérisé en ce qu'**on utilise pour la détection un procédé de restitution d'images, tel qu'un procédé de détection de fluorescence, notamment un procédé basé sur la microscopie à foyer commun ou conventionnelle.

10. Procédé selon une des revendications de 1 à 9, **caractérisé en ce que** le nombre de cellules identifiées comme apoptotiques est normé sur le nombre total de cellules.

11. Procédé selon une des revendications de 1 à 10, **caractérisé en ce que** la détection s'effectue après un intervalle de quelques heures ou après de plus grands intervalles de temps.

12. Procédé selon une des revendications de 1 à 11, **caractérisé en ce qu'**en tant que marqueur, on utilise de l'annexine V en présence de calcium dans une plage de concentration allant de 0,1 à 30 mM, de préférence de 1 à 10 mM.

13. Procédé selon une des revendications de 1 à 12 destiné à rechercher des substances actives apoptotiques.

14. Utilisation d'un kit comportant au moins un agent cytostatique et au moins un marqueur dont l'aptitude à se lier spécifiquement à la phosphatidylsérine est détectable, dans lequel ces agents sont sous une forme sèche, en solution ou contenues dans des substances matrices, dans un procédé selon une des revendications de 1 à 13 pour déterminer la chimiosensibilité des cellules vis à vis d'au moins une substance.
